# FASCICULE DE BREVET EUROPEEN

(11) **EP 0 520 865 B1**
(45) Date de publication et mention de la délivrance du brevet: **27.03.1996**
(21) Numéro de dépôt: 92401719.7
(22) Date de dépôt: 19.06.1992
(51) Int. Cl.: A01N 37/06, A01N 37/02, A61K 31/23, A61K 31/19

(54) **Utilisation de dérivés d'acide pour la fabrication d'un médicament pédiculicide**
Verwendung von Säurederivaten zur Herstellung eines Medikamentes gegen Läuse
Use of acid derivatives for the preparation of a pediculicidal medicament

(30) Priorité: 27.06.1991 FR 9107974; 20.05.1992 FR 9206149
(43) Date de publication de la demande: 30.12.1992
(73) Titulaire: ELF ATOCHEM S.A., F-92800 Puteaux (FR); DELTA AGRO INDUSTRIES, F-75015 Paris (FR)
(72) Inventeur: Caupin, Henri-Jean, F-78000 Versailles (FR); Menassa, Aimé, F-75016 Paris (FR)
(74) Mandataire: Hirsch, Marc-Roger

(56) Documents cités:
- EP-A- 0 279 523
- EP-A- 0 392 806
- BE-A- 544 335
- DE-A- 1 792 467
- DE-A- 2 263 509
- FR-A- 2 377 154
- GB-A- 2 222 774
- JP-A- 5 839 604
- US-A- 3 702 360
- US-A- 5 017 615
- D.E.H.FREAR 'CHEMISTRY OF INSECTICIDES AND FUNGICIDES' Avril 1945 , D.VAN NOSTRAND , NEW YORK, US
- CHEMICAL ABSTRACTS, vol. 98, no. 25, 20 Juin 1983, Columbus, Ohio, US; abstract no. 211570, AGENCY OF INDUSTRIAL SCIENCES AND TECHNOLOGY 'Wool insect controlling agents' page 219 ;colonne 1 ;
- CHEMICAL ABSTRACTS, vol. 50, no. 18, 25 Septembre 1956, Columbus, Ohio, US; abstract no. 13358e, W.F.BARTHEL ET AL. 'Screening tests of esters of piperonyl alcohols as insecticides'

## Description

La présente invention a pour objet une utilisation nouvelle de certains dérivés, ioniques ou non ioniques, de l'acide undécylénique ou de l'acide undécanoïque pour la fabrication d'un médicament pédiculicide. Elle concerne également une composition pharmaceutique nouvelle convenant notamment au traitement des infestations par les parasites pédiculaires.

Les pédiculoses sont provoquées par les poux, parasites exclusifs de l'homme, lesquels sont essentiellement classés en trois espèces:
- Pediculus humanus, variété capitis, ou poux de tête,
- Pediculus humanus, variété corporis, ou poux de corps,
- Phtirius inguinalis ou poux du pubis.

Ces pédiculoses qui restent très courantes sous leurs formes tant endémiques qu'épidémiques, non seulement entraînent des démangeaisons, et les lésions de grattage conséquentes, mais encore les poux sont des vecteurs de certaines maladies, comme le typhus exanthématique et la fièvre récurrente.

Parmi les pédiculicides couramment employés, qui sont en fait des anti-parasitaires polyvalents comparables aux insecticides utilisés dans les traitements en agriculture, sont tout particulièrement employés des composés de la famille des organochlorés ou organophosphorés ou encore des pyrèthres. Ces composés sont présentés sous des formes diverses, notamment sous forme de poudres, de lotions, de shampooings ou d'aérosols. Chacune de ces formes a ses avantages et ses inconvénients. Ainsi, les poudres, a priori moins toxiques et d'emploi commode, nécessitent le port d'une coiffe. Les lotions, d'application aisée et homogène, nécessitent contact prolongé et rinçage; de plus, elles peuvent, du fait de leur écoulement, produire une irritation des muqueuses. Les shampooings peuvent être à la base d'intolérances dues au détergent qu'ils contiennent et nécessitent un temps de contact relativement important. Les aérosols possèdent une action dissolvante des poux et des lentes mais leur emploi ne permet pas une application contrôlée et de plus peut produire certaines irritations oculaires.

Les compositions généralement utilisés sont à base des composés suivants:
- DDT (dichlorodiphényltrichloroéthane) qui est irritant, provoque des modifications de la formule et de la numération sanguine ainsi que des fonctions hépatiques et du système nerveux central;
- hexachlorohexane et lindane qui sont irritants pour la peau, les muqueuses et les yeux et pour lesquels ont été signalées certaines intoxications (vomissements, diarrhées, hyperexcitabilité, convulsions, oedème aigu du poumon, comas);
- pyréthrines naturelles ou de synthèse (telles bioallethrine, bioresméthrine, néopyramine, sumithrine, D-phénothrine) qui sont allergisantes et quelquefois irritantes;
- benzoate de benzyle et malathion irritants pour les yeux, le cuir chevelu.

Les incidents indiqués sont relativement rares, pratiquement inexistants dans les conditions normales d'utilisation, et se sont essentiellement produits chez des nourrissons. Ils se sont cependant produits et impliquent donc des risques graves. A côté de ces risques majeurs, les produits couvrants conduisent souvent à des irritations cutanées.

Les produits existants ont en outre l'inconvénient d'être peu ovicides, c'est-à-dire n'être que faiblement actifs sur les lentes des poux. En fait, les compositions actuellement employées ne permettent pas une lutte efficace contre les infestations de parasites pédiculaires, tant adultes qu'à l'état de lentes.

Le document GB-A-2 222 774 (D1) décrit des compositions pédiculicides consistant en un mélange d'esters d'un sucre avec un acide gras en contenant de 10 à 20 atomes de carbone en association avec un dérivé de cellulose hydrophile. Les exemples ne mentionnent cependant que des esters de saccharose avec des acides palmitique et stéarique.

Le document CAS-1956-13358e (D2) décrit une pluralité d'esters d'alcool pipéronylique, dont 3 esters d'acide undécylénique avec ledit alcool pipéronylique. Certains des composés testés dans ce document sont indiqués comme possédants un activité pédiculicide.

Le but que se propose de résoudre l'invention est de mettre sur le marché une composition pédiculicide qui soit active sur les lentes et les adultes, qui en plus soit à base de produits naturels et qui, en outre, présente une innocuité vis-à-vis des patients, hommes et animaux supérieurs. Les pédiculoses provoquées par une infestation pédiculaires sont donc ainsi évitées.

La présente invention a pour objet l'utilisation pour la fabrication d'un médicament pédiculicide pour le traitement des infestations par des parasites pédiculaires d'une quantité efficace d'un ou plusieurs dérivés lipophiles non-ioniques et/ou hydrophiles ioniques de l'acide undécylénique ou de l'acide undécanoïque.

Selon un mode d'utilisation, les dérivés lipophiles non-ioniques consistent en des esters de cet acide undécylénique ou de cet acide undécanoïque avec un monoalcool en C₁-C₁₆ ne contenant pas d'autre fonction OH que celle qui réagit avec la fonction COOH de l'acide undécylénique ou undécanoïque.

Selon un autre mode d'utilisation, le monoalcool comporte de 1 à 6 atomes de carbone.

Selon encore un autre mode d'utilisation, le monoalcool comporte de 1 ou 2 atomes de carbone.

L'acide undécylénique qui présente la formule:

CH₂=CH(CH₂)₈COOH

et ses sels métalliques (sels de Ca, Cu, Na, Zn) ainsi que l'ester méthylique sont connus dans des applications à titre de bactéricides et de fongicides topiques. Notamment, l'acide undécylénique a déjà été utilisé pour le traitement des infections fongiques de la peau, notamment du cuir chevelu et, plus spécifiquement, pour le traitement des dermatophytoses dues aux teignes. Par ailleurs, selon les travaux menés sur les cheveux, la présence d'acide undécylénique a été mise en évidence parmi les acides gras des cheveux. Aucune corrélation entre la proportion d'acide undécylénique et la lutte contre les poux n'a été mise en évidence au cours de ces travaux.

L'acide undécanoïque qui présente la formule :

CH₃-(CH₂)₉-COOH

n'est pas connu dans des applications pharmacologiques.

La présente invention a également pour objet une composition pharmaceutique nouvelle, et notamment une telle composition convenant au traitement des pédiculoses, caractérisée en ce qu'elle contient des dérivés lipophiles non-ioniques consistant en des esters de l'acide undécylénique ou de l'acide undécanoïque avec un monoalcool en C₁-C₁₆ ne contenant pas d'autre fonction OH que celle qui réagit avec la fonction COOH de l'acide undécylénique ou undécanoïque.

Selon une forme d'exécution de ladite composition suivant l'invention, le monoalcool comporte de 1 à 6 atomes de carbone et, de préférence, 1 ou 2 atomes de carbone.

Selon une autre forme d'exécution de ladite composition suivant l'invention, lesdites compositions contiennent un solvant organique desdits esters de l'acide undécylénique.

Selon encore une autre forme d'exécution de ladite composition suivant l'invention, le solvant est un mélange 30-70/70-30 alcool/acétone, et de préférence 50/50 alcool/acétone.

Selon un autre mode de réalisation de ladite composition, lesdits dérivés hydrophiles ioniques consistent en des sels métalliques, de préférence des sels des métaux alcalins ou alcalino-terreux.

Les sels métalliques sont, par exemple, les sels de Zn, Cu, Na, Ca, K, et autres.

Le sel particulièrement préféré est le sel de sodium.

De façon générale, le médicament peut contenir des dosages variables du principe actif, en fonction de l'infestation à traiter, de facteurs personnels liées au patient, et autres. Cependant, selon une forme d'exécution de l'invention, ledit médicament contient ledit dérivé lipophile ou hydrophile en une proportion pondérale de l'ordre de 2 à 20% exprimée par rapport au poids dudit médicament.

L'invention se rapporte en outre à l'application pharmacologique des esters précités pour le traitement des pédiculoses.

D'autres buts et avantages de la présente invention apparaîtront à la lecture de la description suivante et des exemples donnés à titre illustratif et nullement limitatif.

Dans ces exemples, sauf indication contraire, les proportions sont exprimées en proportions pondérales.

Les traitements mis en oeuvre consistent en une application des compositions, solutions ou suspensions employées à l'aide de compresses appliquées par tamponnement, lesdites compresses ayant été largement imbibées de ces compositions par immersion.

### EXEMPLE 1

Un test blanc effectué avec de l'eau uniquement conduit aux résultats consignés dans le tableau I ci-dessous.

**TABLEAU I**

| | nombre de lentes | % |
|---|---|---|
| départ | 108 | 100 |
| éclosion | 66 | 61 |
| survie | 64 | 59 |

### EXEMPLE 2

Cet exemple donné à titre témoin a porté sur l'emploi d'un dérivé hydrophile non-ionique de l'acide undécylénique consistant en un mono-undécylénate de glycérol mis en une solution aqueuse contenant 30% de cet ester.

Un test effectué à l'aide de la composition ainsi obtenue conduit aux résultats suivants:

**TABLEAU II**

| | nombre de lentes | % |
|---|---|---|
| départ | 89 | 100 |
| éclosion | 54 | 61 |
| survie | 53 | 60 |

Le même test effectué en employant le di-undécylénate de glycérol conduit à des résultats pratiquement identiques en pourcentage.

### EXEMPLE 3

Un test effectué dans des conditions identiques avec une solution aqueuse contenant 30% d'undécylénate de PEG 400 conduit aux résultats consignés dans le tableau suivant:

**TABLEAU III**

| | nombre de lentes | % |
|---|---|---|
| départ | 103 | 100 |
| éclosion | 55 | 53 |
| survie | 53 | 51 |

Les résultats auxquels l'emploi d'une solution contenant un dérivé hydrophile non-ionique conduit montrent qu'ils ne permettent pas d'obtenir des résultats meilleurs que ceux auquel conduit un simple rinçage à l'eau comme indiqué à l'exemple 1.

### EXEMPLE 4

Dans cet exemple, est employé à titre de témoin une solution alcool/acétone 50/50.

L'application de cette solution, dans les conditions indiquées, conduit aux résultats suivants:

**TABLEAU IV**

| | nombre de lentes | % |
|---|---|---|
| départ | 116 | 100 |
| éclosion | 55 | 47 |
| survie | 52 | 45 |

Une telle solution conduit à des résultats inintéressants dans la mesure où 50% uniquement des lentes sont détruites, de façon comparable aux exemples précédents.

### EXEMPLES 5 ET 6

On emploie un dérivé lipophile consistant en undécylénate de méthyle (exemple 5) et undécylénate d'éthyle (exemple 6) en solution à 10% dans un mélange 50/50 alcool/acétone.

On obtient les résultats consignés dans le tableau V ci-dessous.

**TABLEAU V**

| | undécylénate de méthyle | | undécylénate d' éthyle | |
|---|---|---|---|---|
| | nb de lentes | % | nb de lentes | % |
| départ | 92 | 100 | 92 | 100 |
| éclosion | 0 | 0 | 6 | 6,5 |
| survie | 0 | 0 | 0 | 0 |

Les dérivés selon la présente invention sont donc remarquablement actifs.

### EXEMPLE 7

On emploie dans ce test un dérivé ionique hydrophile consistant en le sel de sodium de l'acide undécylénique. Le test est mis en oeuvre d'une part sur 137 lentes âgées de 2 à 5 jours (tableau VI) et sur 25 poux adultes âgés de 18 à 20 jours (tableau VII). Par ailleurs, dans le test sur les lentes, on a mesuré le pourcentage d'insectes qui se nourrissent -estimation de la survie-. Les témoins sont constitués par un témoin eau et un témoin sec. Dans les tableaux, Nb est l'effectif de départ, % représente le % d'eclosion ou de survie, respectivement.

**TABLEAU VI**

| | produit | | témoin eau | | témoin sec | |
|---|---|---|---|---|---|---|
| | Nb | % | Nb | % | Nb | |
| Eclosion | 137 | 0 | 74 | 42 | 83 | 73,5 |
| Vivants | - | - | 31 | 87 | 61 | 82 |
| Eclosion = % d'éclosion, Vivants = % d'insectes éclos qui se nourrissent. | | | | | | |

**TABLEAU VII**

| | produit | | | témoins (eau et sec) | | |
|---|---|---|---|---|---|---|
| | Nb | % | vivants | Nb | % | vivants |
| mortalité | 25 | 100 | 0 | 0 | 0 | 25 |

On constate donc de la comparaison des résultats consignés dans les divers tableaux:
- que l'emploi de solutions ou suspensions à base de dérivés hydrophiles de l'acide undécylénique, tels mono-ou di-undécylénate de glycérol ou undécylénate de PEG 400, ne conduit pas à un taux de destruction des lentes et des poux supérieur à celui auquel un rinçage à l'aide d'eau conduit;
- que l'emploi de dérivés lipophiles non-ioniques ou hydrophile ionique de l'acide undécylénique, en solution alcool/acétone ou en solution aqueuse respectivement, conduit à la destruction totale ou pratiquement totale, non seulement des poux, mais encore des lentes.

## Revendications

1. Application d'esters de l'acide undécylénique avec un monoalcool en C₁-C₆ ne contenant pas d'autre fonction OH que celle qui réagit avec la fonction COOH de l'acide undécylénique pour obtenir un médicament destiné au traitement des infestations par des parasites pédiculaires.

2. Application selon la revendication 1, dans laquelle dans les esters le monoalcool comporte de 1 ou 2 atomes de carbone.

3. Application selon la revendication 1 ou 2, dans laquelle ledit médicament contient un solvant organique desdits esters de l'acide undécylénique.

4. Application selon la revendication 3, dans laquelle ledit solvant organique est un mélange 30-70/70-30 alcool/acétone.

5. Application de sels métalliques de l'acide undécylénique pour obtenir un médicament destiné au traitement des infestations par des parasites pédiculaires.

6. Application selon la revendication 5, dans laquelle lesdits sels sont les sels alcalins ou alcalino-terreux.

7. Application selon la revendication 6, dans laquelle le sel est le sel de sodium.

8. Application selon l'une quelconque des revendications 1 à 7, dans laquelle ledit médicament contient ledits esters ou sels en des proportions pondérales de l'ordre de 2 à 20%, exprimées par rapport au poids dudit médicament.

9. Application selon l'une quelconque des revendications 1 à 8, dans laquelle ledit médicament est sous forme pulvérulente, éventuellement en mélange avec un support solide pharmacologiquement acceptable.

10. Application selon l'une quelconque des revendications 1 à 8, dans laquelle ledit médicament est sous forme liquide, éventuellement en mélange avec un support liquide pharmacologiquement acceptable.

## Claims

1. The application of esters of undecylenic acid with a C₁ to C₆ monoalcohol containing no other OH function apart from the one reacting with the COOH function of undeclylenic acid for obtaining a medicament for treating infestations by pedicular parasites.

2. Application according to claim 1 characterized in that, in said esters, the monoalcohol contains 1 or 2 carbon atoms.

3. Application according to claim 1 or 2, characterized in that said medicament contains an organic salt for said undecylenic acid esters.

4. Application according to claim 3, characterized in that said organic solvent is a 30-70/70-30 alcohol/acetone mixture.

5. The application of undecylenic acid metal salts to the obtaining of a medicament for treatment of infestations caused by pedicular parasites

6. Application according to claim 5, characterized in that the said salts are the alkali or alkaline earth salts.

7. Application according to claim 6, characterized in that the salt is the sodium salt.

8. Application according to any one of claims 1 to 7, characterized in that said medicament contains said esters or salts in an amount by weight of about 2 to 20% based on the weight of said medicament.

9. Application according to any one of claims 1 to 8, in which said medicament is in powder form optionally mixed with a pharmacologically acceptable support.

10. Application according to any one of claims 1 to 8, characterized in that said medicament is in a liquid form optionally mixed with a pharmacologically acceptable liquid support.

## Patentansprüche

1. Verwendung von Estern der Undecensäure mit einem C₁- bis C₆-Monoalkohol, der keine andere OH-Funktion als diejenige enthält, die mit der COOH-Funktion der Undecensäure reagiert, um ein Medikament zur Behandlung von durch Läuseparasiten hervorgerufenen Infestationen zu erhalten.

2. Verwendung nach Anspruch 1, wobei der Monoalkohol in den Estern 1 bis 2 Kohlenstoffatome enthält.

3. Verwendung nach Anspruch 1 oder 2, wobei das Medikament ein organisches Lösemittel für die Undecensäureester enthält.

4. Verwendung nach Anspruch 3, wobei das organische Lösemittel eine Alkohol/Aceton-Mischung von 30-70/70-30 ist.

5. Verwendung von Metallsalzen der Undecensäure, um ein Medikament zur Behandlung von durch Läuseparasiten hervorgerufenen Infestationen zu erhalten.

6. Verwendung nach Anspruch 5, wobei die Salze Alkali- oder Erdalkalisalze sind.

7. Verwendung nach Anspruch 6, wobei das Salz ein Natriumsalz ist.

8. Verwendung nach einem der Ansprüche 1 bis 7, wobei das Medikament die Ester oder die Salze in einer Menge von etwa 2 bis 20 Gew.-% enthält, bezogen auf das Gewicht des Medikaments.

9. Verwendung nach einem der Ansprüche 1 bis 8, wobei das Medikament in feinpulvriger Form, gegebenenfalls in Mischung mit einem festen, pharmakologisch verträglichen Träger, vorliegt.

10. Verwendung nach einem der Ansprüche 1 bis 8, wobei das Medikament in flüssiger Form, gegebenenfalls in Mischung mit einem flüssigen, pharmakologisch verträglichen Träger, vorliegt.
